# FASCICULE DE BREVET EUROPEEN

(11) **EP 1 886 661 B1**
(45) Date de publication et mention de la délivrance du brevet: **06.10.2010**
(21) Numéro de dépôt: 07113250.0
(22) Date de dépôt: 26.07.2007
(51) Int. Cl.: A61K 8/41, A61K 8/81, A61Q 5/10

(54) **Composition tinctoriale comprenant un colorant direct et un polymère amphotère comprenant des motifs acrylamide, halogénure de dialkyldiallylammonium et acide carboxylique vinylique**
Färbezusammensetzung enthaltend ein Oxidationsfärbemittel und ein amphoterisches Polymer enthaltend Acrylamideinheiten, Dialkyldiallylammoniumhalid und Vinylcarbonsäure
Tinctorial composition containing a direct dye and an amphoteric polymer comprising units of acrylamide, dialkyldiallylammonium halide and vinyl carboxylic acid

(30) Priorité: 10.08.2006 FR 0607249
(43) Date de publication de la demande: 13.02.2008
(73) Titulaire: L'Oréal, 75008 Paris (FR)
(72) Inventeur: Cottard, François, 92400, Courbevoie (FR); Deconinck, Gautier, 95210, Saint Gratien (FR)
(74) Mandataire: Dossmann, Gérard

(56) Documents cités:
- EP-A1- 0 521 665
- EP-A1- 0 522 755
- WO-A-02/45674
- WO-A-94/06403
- US-A1- 2004 133 996

## Description

La présente invention a pour objet une composition pour la teinture directe des fibres kératiniques, et en particulier des fibres kératiniques humaines telles que les cheveux, comprenant, dans un milieu approprié pour la teinture, au moins un colorant direct et au moins un polymère amphotère particulier.

Il est connu de teindre les fibres kératiniques et en particulier les cheveux humains avec des compositions tinctoriales contenant des colorants d'oxydation, en particulier des précurseurs de colorants d'oxydation et des modificateurs de coloration.

Les précurseurs de colorants d'oxydation, appelés généralement bases d'oxydation, sont initialement des composés incolores ou faiblement colorés qui, associés à des produits oxydants, peuvent donner naissance par un processus de condensation oxydative à des composés colorés et colorants. Ce sont en général des composés tels que les ortho- ou para-phénylènediamines, les ortho- ou para-aminophénols et les bases hétérocycliques.

On sait également que l'on peut faire varier les nuances obtenues avec ces bases d'oxydation en les associant à des coupleurs ou modificateurs de coloration, ces derniers étant généralement choisis parmi les méta-diaminobenzènes, les méta-aminophénols, les méta-diphénols et certains composés hétérocycliques tels que des composés indoliques.

La variété des molécules mises en jeu au niveau des bases d'oxydation et des coupleurs permet l'obtention d'une riche palette de couleurs. La coloration dite "permanente" obtenue grâce à ces colorants d'oxydation est appelée également coloration d'oxydation.

Il est également connu de teindre les fibres kératiniques humaines par une coloration dite directe. Le procédé classiquement utilisé en coloration directe consiste à appliquer sur les fibres kératiniques des colorants directs qui sont des molécules colorées et colorantes ayant une affinité pour les fibres, à laisser poser, puis à rincer les fibres.

Cette coloration peut se faire en l'absence ou en présence d'un agent oxydant tel que l'eau oxygénée et/ou les persels. Dans le dernier cas, on parle de coloration directe éclaircissante.

Il est connu par exemple d'utiliser des colorants directs nitrés benzèniques, anthraquinoniques, nitropyridiniques, des colorants azoïques, xanthéniques, acridiniques aziniques ou des colorants triarylméthaniques.

Les colorations qui en résultent sont des colorations particulièrement chromatiques, et qui sont temporaires ou semi-permanentes. En effet, la nature des interactions qui lient les colorants directs à la fibre kératinique, et leur désorption de la surface et/ou du coeur de la fibre sont responsables de leur moindre puissance tinctoriale et de leur moindre tenue aux lavages ou à la transpiration.

Toutefois, et de manière connue en soi, les compositions de coloration directe ont tendance à tacher le cuir chevelu. Cet effet peu esthétique est perçu comme un inconvénient dans l'utilisation de ces produits de coloration directe, par rapport aux produits de coloration d'oxydation.

Le but de la présente invention est d'obtenir de nouvelles compositions pour la teinture directe des fibres kératiniques, qui ne présentent pas les inconvénients de l'art antérieur. Plus particulièrement, le but de la présente invention est d'obtenir des compositions de teinture directe qui permettent de réduire le tachage du cuir chevelu, et ce sans pour autant altérer la puissance, la chromaticité et la sélectivité de la coloration.

Ce but est atteint avec la présente invention qui a pour objet une composition pour la teinture directe des fibres kératiniques, et en particulier des fibres kératiniques humaines telles que les cheveux, comprenant, dans un milieu approprié pour la teinture, au moins un colorant direct et au moins un polymère amphotère comprenant la répétition de :
(i) un ou plusieurs motifs issus d'un monomère de type acrylamide,
(ii) un ou plusieurs motifs issus d'un monomère de type halogénure de dialkyldiallylammonium, et
(iii) au moins 50 % en moles de un ou plusieurs motifs issus d'un monomère de type acide carboxylique vinylique.

La composition conforme à la présente invention permet de réduire, voire d'éliminer le tachage du cuir chevelu, tout en conservant de bonnes propriétés rhéologiques, et en restant facile d'emploi.

En outre, elle permet d'améliorer les propriétés cosmétiques des cheveux, notamment en ce qui concerne le démêlage et le lissage.

Enfin, les propriétés tinctoriales de cette composition sont très satisfaisantes, tant en ce qui concerne la sélectivité que la chromaticité de la coloration obtenue.

La présente invention a également pour objet un procédé de teinture directe des fibres kératiniques, dans lequel une composition conforme à l'invention est appliquée sur les fibres kératiniques pendant un temps suffisant pour développer la coloration désirée.

La présente invention a aussi pour objet l'utilisation pour la teinture directe des fibres kératiniques de la composition conforme à l'invention.

La présente invention a enfin pour objet l'utilisation, dans une composition de teinture directe des fibres kératiniques, d'un polymère amphotère tel que décrit dans la présente demande, afin de réduire le tachage du cuir chevelu.

A moins d'une indication différente, les bornes des gammes de valeurs qui sont données dans le cadre de la présente invention sont incluses dans ces gammes.

Selon un mode de réalisation particulier, les motifs issus d'un monomère de type acrylamide du polymère amphotère utile dans le cadre de l'invention sont des motifs de structure (I) suivante : 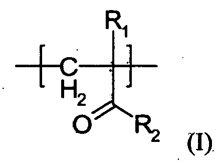 dans laquelle :
- R₁ désigne H ou CH₃,
- R₂ est choisi parmi un radical amino, diméthylamino, tert-butylamino, dodécylamino, ou -NH-CH₂OH.

De préférence, le polymère amphotère de l'invention ne comprend la répétition que d'un seul motif de formule (I).

Le motif issu d'un monomère de type acrylamide de formule (I) dans laquelle R₁ désigne H et R₂ est un radical amino est particulièrement préféré. Il correspond au monomère acrylamide proprement dit.

Selon un autre mode de réalisation particulier de l'invention, les motifs issus d'un monomère de type halogénure de dialkyldiallylammonium du polymère amphotère sont des motifs de structure (II) suivante : 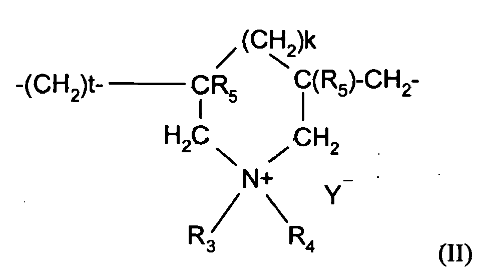 dans laquelle :
- k et t sont égaux à 0 ou 1, la somme k + t étant égale à 1 ;
- R₅ désigne un atome d'hydrogène ou un radical méthyle ;
- R₃ et R₄, indépendamment l'un de l'autre, désignent un groupement alkyle ayant de 1 à 4 atomes de carbone, un groupement hydroxyalkyle dans lequel le groupement alkyle a de préférence 1 à 5 atomes de carbone, un groupement amidoalkyle(C₁-C₄), ou R₃ et R₄ forment conjointement avec l'atome d'azote auquel ils sont rattachés, des groupements hétérocycliques, tels que pipéridinyle ou morpholinyle ; R₃ et R₄ indépendamment l'un de l'autre désignent de préférence un groupement alkyle ayant de 1 à 4 atomes de carbone ;
- Y⁻ est un anion tel que bromure, chlorure, acétate, borate, citrate, tartrate, bisulfate, bisulfite, sulfate, phosphate.

Parmi ces motifs issus d'un monomère de type halogénure de dialkyldiallylammonium, on préfère ceux issus du monomère chlorure de diméthyldiallylammonium pour lesquels R₅ désigne un atome d'hydrogène et R₃ et R₄ désignent un radical méthyle, Y⁻ désignant un anion chlorure.

Selon un mode de réalisation particulier de l'invention, les motifs issus d'un monomère de type acide carboxylique vinylique du polymère amphotère sont choisis parmi les motifs de formule (III) : 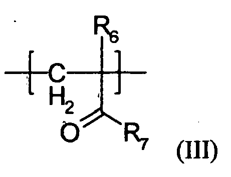 dans laquelle :
- R₆ désigne H ou CH₃,
- R₇ désigne un radical hydroxyle ou un radical -NH-C(CH₃)₂-CH₂-SO₃H.

Les motifs préférés correspondent aux monomères acide acrylique, acide méthacrylique et acide 2-acrylamido 2-méthyl propane sulfonique.

De préférence, le motif issu d'un monomère de type acide carboxylique vinylique est celui issu de l'acide acrylique pour lequel R₆ désigne un atome d'hydrogène et R₇ désigne un radical hydroxyle.

Selon l'invention, le ou les polymères amphotères comprennent au moins 50 % en moles de motifs issus d'un monomère de type acide carboxylique vinylique.

De préférence, ils comprennent de 50 à 90 % en moles de motifs issus d'un monomère de type acide carboxylique vinylique, de manière plus préférée de 50 à 75 % en moles.

Les teneurs des deux autres motifs peuvent avantageusement être les suivantes :
- de 1 à 40 %, préférentiellement de 5 à 25 % en moles de motifs issus d'un monomère de type acrylamide;
- de 1 à 40 %, préférentiellement de 5 à 25 % en moles de motifs issus d'un monomère de type halogénure de dialkyldiallylammonium.

Le ou les polymères amphotères utiles dans le cadre de l'invention peuvent également comprendre des motifs additionnels, différents des motifs issus d'un monomère de type acrylamide, halogénure de dialkyldiallylammonium, et acide carboxylique vinylique, du moment qu'ils comprennent au moins 50 % en moles de motifs issus d'un monomère de type acide carboxylique vinylique.

Comme exemple de polymères à motifs issus d'un monomère de type (i) acrylamide, (ii) halogénure de dialkyldiallylammonium, et (iii) acide carboxylique vinylique, on peut citer notamment les terpolymères acrylamide / chlorure de diméthyldiallylammonium / acide acrylique, répertoriés dans le dictionnaire C.T.F.A. International Cosmetic Ingredient Dictionary, 10ème édition 2004, sous la dénomination "Polyquaternium 39". Les polymères selon l'invention peuvent ainsi être choisis parmi les Polyquaterniums 39 contenant au moins 50% en moles de motifs issus de l'acide acrylique, tel que par exemple le produit commercialisé sous la dénomination MERQUAT 3333 par la société NALCO.

Le polymère amphotère selon l'invention peut être préparé de manière classique, par polymérisation à partir de ses différents monomères, selon des techniques connues de l'homme du métier et notamment par polymérisation radicalaire.

Le ou les polymères amphotères utiles dans le cadre de l'invention sont généralement présents en quantité comprise entre 0,1 et 10 % en poids, de préférence entre 0,5 et 5 % en poids, et plus particulièrement entre 1 et 4 % en poids par rapport au poids total de la composition.

La composition conforme à l'invention comprend au moins un colorant direct. Les colorants directs utilisables dans les compositions selon la présente demande peuvent être choisis parmi les colorants directs acides, basiques ou neutres.

Les colorants directs utilisables selon l'invention sont choisis de préférence parmi les colorants directs nitrés benzéniques neutres, acides ou cationiques, les colorants directs azoïques, azométhiniques ou méthiniques neutres, acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs nitrés benzéniques utilisables selon l'invention, on peut citer de manière non limitative les composés suivants:
- 1,4-diamino-2-nitrobenzène
- 1-amino-2 nitro-4-β- hydroxyéthylaminobenzène
- 1-amino-2 nitro-4-bis(β-hydroxyéthyl)-aminobenzène
- 1,4-Bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1 -amino-3-méthyl-4-β-hydroxyéthylamino-6-nitrobenzène
- 1 -amino-2-nitro-4-β-hydroxyéthylamino-5-chlorobenzène
- 1,2-diamino-4-nitrobenzène
- 1-amino-2-β-hydroxyéthylamino-5-nitrobenzène
- 1,2-bis-(β-hydroxyéthylamino)-4-nitrobenzène
- 1 -amino-2-tris-(hydroxyméthyl)-méthylamino-5-nitrobenzène
- 1-hydroxy-2-amino-5-nitrobenzène
- 1-hydroxy-2-amino-4-nitrobenzène
- 1-hydroxy-3-nitro-4-aminobenzène
- 1-hydroxy-2-amino-4,6-dinitrobenzène
- 1-β-hydroxyéthyloxy-2-β-hydroxyéthylamino-5-nitrobenzène
- 1 -méthoxy-2-β-hydroxyéthylamino-5-nitrobenzène
- 1- β-hydroxyéthyloxy-3-méthylamino-4-nitrobenzène
- 1 -β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-hydroxy-2-chloro-6-éthylamino-4-nitrobenzène
- 1-hydroxy-2-chloro-6-amino-4-nitrobenzène
- 1-hydroxy-6-bis-(β-hydroxyéthyl)-amino-3-nitrobenzène
- 1-β -hydroxyéthylamino-2-nitrobenzène
- 1-hydroxy-4-β-hydroxyéthylamino-3-nitrobenzène.

Parmi les colorants directs azoïques azométhiniques ou méthiniques utilisables selon l'invention on peut citer par exemple les colorants azoïques cationiques décrits dans les demandes de brevets WO 95/15144, WO-95/01772 et EP-714954 dont le contenu fait partie intégrante de l'invention.

Parmi ces composés on peut tout particulièrement citer les colorants suivants:
- chlorure de 1,3-diméthyl-2-[[4-(diméthylamino)phényl]azo]-1H-imidazolium,
- chlorure de 1,3-diméthyl-2-[(4-aminophényl)azo]-1H-imidazolium,
- méthylsulfate de 1-méthyl-4-[(méthylphényl-hydrazono)méthyl]-pyridinium.

On peut aussi citer les colorants décrits dans les demandes de brevet EP 1 170 000, EP 1 166 753, EP 1 166 754, EP 1 170 001, EP 1 025 834, les parties de ces demandes concernant les colorants directs azoïques, azométhiniques ou méthiniques font partie intégrante de la présente demande.

On peut également citer parmi les colorants directs azoïques les colorants suivants, décrits dans le COLOUR INDEX INTERNATIONAL, 3e édition :
- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques on peut citer les colorants suivants :
- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99
ainsi que les composés suivants :
- 1-N-méthylmorpho liniumpropylamino-4-hydroxyanthraquinone
- 1-aminopropylamino-4-méthylaminoanthraquinone
- 1-aminopropylaminoanthraquinone
- 5-β-hydroxyéthyl-1,4-diaminoanthraquinone
- 2-aminoéthylaminoanthraquinone
- 1,4-bis-(β,γ-dihydroxypropylamino)-anthraquinone.
Parmi les colorants aziniques on peut citer les composés suivants :
- Basic Blue 17
- Basic Red 2.

Parmi les colorants triarylméthaniques utilisables selon l'invention, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques utilisables selon l'invention, on peut citer les composés suivants :
- 2-β-hydroxyéthylamino-5-[bis-(β-4'-hydroxyéthyl)amino]anilino-1,4-benzoquinone
- 2-(3 -hydroxyéthylamino-5-(2'-méthoxy-4'-amino)anilino-1,4-benzoquinone
- 3-N(2'-chloro-4'-hydroxy)phényl-acétylamino-6-méthoxy-1,4-benzoquinone imine
- 3-N(3'-chloro-4'-méthylamino)phényl-uréido-6-méthyl-1,4-benzoquinone imine
- 3-[4'-N-(éthyl,carbamylméthyl)-amino]-phényl-uréido-6-méthyl-1,4-benzoquinone imine.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

De préférence, la composition selon la présente invention comprend au moins un colorant direct cationique porteur d'une charge permanente dans la molécule.

La composition selon la présente invention comprend généralement une quantité totale de colorant(s) direct(s) allant de 0,001% à 20% en poids, de préférence de 0,05% à 10% en poids, par rapport au poids total de la composition.

La composition conforme à la présente invention peut également comprendre au moins un agent oxydant, notamment lorsque l'on veut obtenir une coloration directe éclaircissante.

Un tel agent oxydant est alors choisi de préférence dans le groupe formé par le peroxyde d'hydrogène, le peroxyde d'urée, les bromates ou ferricyanures de métaux alcalins, les persels tels que les perborates et les persulfates.

L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.

On peut également utiliser à titre d'agent oxydant une ou plusieurs enzymes d'oxydoréduction telles que les laccases, les peroxydases et les oxydoréductases à 2 électrons (telles que l'uricase), éventuellement en présence de leur donneur ou cofacteur respectif.

Le milieu approprié pour la teinture appelé aussi support de teinture est un milieu cosmétique généralement constitué par de l'eau ou par un mélange d'eau et d'au moins un solvant organique acceptable sur le plan cosmétique. A titre d'exemples de solvants organiques, on peut notamment citer les alcools tels que l'alcool éthylique, l'alcool isopropylique, l'alcool benzylique, et l'alcool phényléthylique, ou les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol. Les solvants peuvent alors être présents dans des concentrations comprises entre environ 0,5 et 20 % et, de préférence, entre environ 2 et 10 % en poids par rapport au poids total de la composition.

La composition conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, autres que les polymères amphotères selon l'invention, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci-dessus sont en général présents en quantité comprise pour chacun d'eux entre 0,01 et 20 % en poids par rapport au poids de la composition tinctoriale.

Bien entendu, l'homme de l'art veillera à choisir le ou les éventuels composés complémentaires mentionnés ci-avant, de manière telle que les propriétés avantageuses attachées intrinsèquement aux compositions selon l'invention ne soient pas, ou substantiellement pas, altérées par la ou les adjonctions envisagées.

Le pH de la composition conforme à l'invention est généralement compris entre 3 et 12 environ, de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxyalkylamines et les éthylènediamines oxyéthylénées et/ou oxypropylénées, les hydroxydes de sodium ou de potassium et les composés de formule suivante : 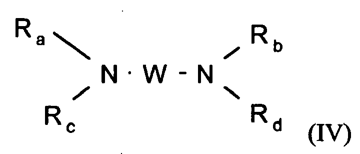 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

Parmi les agents acidifiants, on peut citer, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de teinture des fibres kératiniques selon la présente invention est un procédé dans lequel on applique sur lesdites fibres la composition selon l'invention telle que définie précédemment. Après un temps de pose variant généralement de 1 à 60 minutes environ, de préférence 5 à 45 minutes environ, les fibres kératiniques sont rincées, lavées au shampooing, rincées à nouveau puis séchées.

Selon un mode de réalisation particulier, par exemple lorsque l'on souhaite procéder à une teinture éclaircissante, la composition selon la présente invention peut être mélangée, de préférence au moment de l'emploi, à une composition contenant, dans un milieu approprié pour la teinture, au moins un agent oxydant. Le mélange obtenu est ensuite appliqué sur les fibres kératiniques. Après un temps de pose variant généralement de 1 à 60 minutes environ, de préférence 5 à 45 minutes environ, les fibres kératiniques sont rincées, lavées au shampooing, rincées à nouveau puis séchées.

L'invention a aussi pour objet un dispositif à plusieurs compartiments ou "kit" de teinture dans lequel un premier compartiment contient une composition tinctoriale selon la présente invention à l'exception de l'agent oxydant, et un deuxième compartiment contient un agent oxydant. Ce dispositif peut être équipé d'un moyen permettant de délivrer sur les cheveux le mélange souhaité, tel que les dispositifs décrits dans le brevet FR-2 586 913 au nom de la demanderesse.

La présente invention a également pour objet l'utilisation pour la teinture directe des fibres kératiniques, et en particulier des fibres kératiniques humaines telles que les cheveux, d'une composition tinctoriale telle que définie précédemment.

Les exemples qui suivent sont destinés à illustrer l'invention sans pour autant en limiter la portée.

Dans ces exemples, toutes les quantités sont indiquées en pour cent en poids de matière active (M.A.) par rapport au poids total de la composition, sauf indication contraire.

### Exemple 1 comparatif:

On a préparé deux compositions de teinture à partir des composés suivants (composition A comparative, composition B selon l'invention):

| **Composition** | **A** | **B** |
|---|---|---|
| amide grasse commercialisé sous la dénomination CHIMEXANE HA par la société CHIMEX | 9 | 9 |
| alcool laurique oxyéthyléné à 12 OE commercialisé sous la dénomination REWOPAL 12 par la société GOLDSCHMIDT | 11 | 11 |
| monostéarate de glycol | 1,5 | 1,5 |
| tensioactif cationique de type ammonium quaternaire commercialisé sous la dénomination CHIMEXANE CL par la société CHIMEX | 4,7 | 4,7 |
| cellulose épaisissante commercialisée sous la dénomination BLANOSE 7M8SF par la société AQUALON | 0,3 | 0,3 |
| gomme de xanthane commercialisée sous la dénomination RHODICARE XC par la société RHODIA | 0,3 | 0,3 |
| acide lactique | qs pH 6.7 | qs pH 6.7 |
| monoéthanolamine | qs pH 6.7 | qs pH 6.7 |
| polymère acrylamide / chlorure de diallyldiméthyl ammonium / acide acrylique (Merquat 3333 de Nalco) | - | 2,5 M.A. |
| 1-hydroxy-3-nitro-4-amino-benzène | 0,35 | 0,35 |
| 1-hydroxy-3-nitro-4-beta-hydroxyéthylamino-benzène | 0,33 | 0,33 |
| 1-(beta-hydroxyéthylamino)-2-nitro-4-aminobenzène | 0,02 | 0,02 |
| chlorhydrate de 1-méthoxy-3(beta-aminoéthyl)amino-4-nitrobenzène | 0,02 | 0,02 |
| 2-[4-[(2-aminoéthyl)amino]-3-nitrophénoxy]éthanol | 0,04 | 0,04 |
| parfum | 1 | 1 |
| eau | qsp 100 | qsp 100 |

Les pH des compositions ainsi obtenues sont de 6.7
Ces compositions sont appliquées sur des cheveux gris à 90 % de blancs pendant un temps de pose de 30 minutes à température ambiante.
Les cheveux sont ensuite rincés, lavés avec un shampooing standard, puis rincés à l'eau et séchés.
On obtient pour chacune des compositions A et B une nuance dorée. La nuance obtenue avec la composition B est peu sélective et chromatique. Les cheveux sont doux et se démêlent bien.
On observe que la composition B tâche moins le cuir chevelu que la composition A.

## Revendications

1. Composition pour la teinture directe des fibres kératiniques comprenant, dans un milieu approprié pour la teinture, au moins un colorant direct et au moins un polymère amphotère comprenant la répétition de :
(i) un ou plusieurs motifs issus d'un monomère de type acrylamide,
(ii) un ou plusieurs motifs issus d'un monomère de type halogénure de dialkyldiallylammonium, et
(iii) au moins 50 % en moles de un ou plusieurs motifs issus d'un monomère de type acide carboxylique vinylique.

2. Composition selon la revendication 1 dans laquelle les motifs issus d'un monomère de type acrylamide sont des motifs de structure (I) suivante : 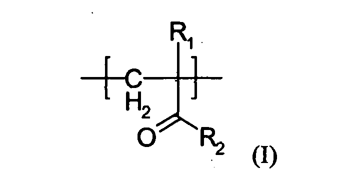 dans laquelle :
- R₁ désigne H ou CH₃,
- R₂ est choisi parmi un radical amino, diméthylamino, tert-butylamino, dodécylamino, ou -NH-CH₂OH.

3. Composition selon la revendication 2 dans laquelle R₁ désigne H et R₂ est un radical amino.

4. Composition selon l'une quelconque des revendications précédentes dans laquelle les motifs issus d'un monomère de type halogénure de dialkyldiallylammonium sont des motifs de structure (II) suivante : 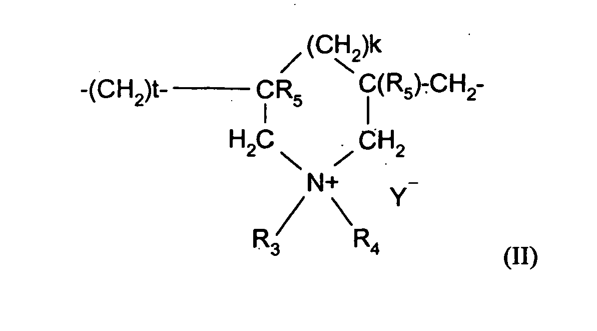 dans laquelle :
- k et t sont égaux à 0 ou 1, la somme k + t étant égale à 1 ;
- R₅ désigne un atome d'hydrogène ou un radical méthyle ;
- R₃ et R₄, indépendamment l'un de l'autre, désignent un groupement alkyle ayant de 1 à 4 atomes de carbone, un groupement hydroxyalkyle dans lequel le groupement alkyle a de 1 à 5 atomes de carbone, un groupement amidoalkyle(C₁-C₄), ou R₃ et R₄ forment conjointement avec l'atome d'azote auquel ils sont rattachés, des groupements hétérocycliques ;
- Y⁻ est un anion bromure, chlorure, acétate, borate, citrate, tartrate, bisulfate, bisulfite, sulfate ou phosphate.

5. Composition selon la revendication 4 dans laquelle les motifs issus d'un monomère de type halogénure de dialkyldiallylammonium sont des motifs de formule (II) dans laquelle R₅ désigne un atome d'hydrogène et R₃ et R₄ désignent un radical méthyle, Y⁻ désignant un anion chlorure.

6. Composition selon l'une quelconque des revendications précédentes dans laquelle les motifs issus d'un monomère de type acide carboxylique vinylique sont choisis parmi les motifs de formule (III) : 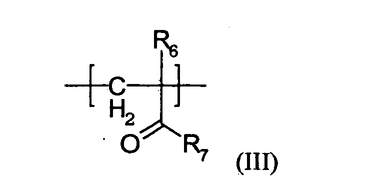 dans laquelle:
- R₆ désigne H ou CH₃,
- R₇ désigne un radical hydroxyle ou un radical -NH-C(CH₃)₂-CH₂-SO₃H.

7. Composition selon la revendication 6 dans laquelle le motif issu d'un monomère de type acide carboxylique vinylique est celui pour lequel R₆ désigne un atome d'hydrogène et R₇ désigne un radical hydroxyle.

8. Composition selon l'une quelconque des revendications précédentes dans laquelle le ou les polymères amphotères comprennent de 50 à 90 % en moles de motifs issus d'un monomère de type acide carboxylique vinylique.

9. Composition selon l'une quelconque des revendications précédentes dans laquelle le ou les polymères amphotères comprennent de 1 à 40 % en moles de motifs issus d'un monomère de type acrylamide.

10. Composition selon l'une quelconque des revendications précédentes dans laquelle le ou les polymères amphotères comprennent de 1 à 40 % en moles de motifs issus d'un monomère de type halogénure de dialkyldiallylammonium.

11. Composition selon l'une quelconque des revendications précédentes dans laquelle le ou les polymères amphotères sont choisis parmi les terpolymères acrylamide / chlorure de diméthyldiallylammonium / acide acrylique contenant au moins 50 % en moles de motifs issus de l'acide acrylique.

12. Composition selon l'une quelconque des revendications précédentes dans laquelle le ou les polymères amphotères sont présents en quantité comprise entre 0,1 et 10 % en poids par rapport au poids total de la composition.

13. Composition selon l'une quelconque des revendications précédentes dans laquelle le ou les colorants directs sont choisis parmi les colorants directs acides, basiques ou neutres.

14. Composition selon la revendication 13 dans laquelle le ou les colorants directs sont choisis parmi les colorants directs nitrés benzéniques neutres, acides ou cationiques, les colorants directs azoïques, azométhiniques ou méthiniques neutres, acides ou cationiques, les colorants directs quinoniques neutres, acides ou cationiques, les colorants directs aziniques, les colorants directs triarylméthaniques, les colorants directs indoaminiques et les colorants directs naturels.

15. Composition selon l'une quelconque des revendications précédentes, comprenant au moins un colorant direct cationique porteur d'une charge permanente dans la molécule.

16. Composition selon l'une quelconque des revendications précédentes, comprenant une quantité totale de colorant(s) direct(s) allant de 0,001 % à 20 % en poids par rapport au poids total de la composition.

17. Composition selon l'une quelconque des revendications précédentes, comprenant au moins un agent oxydant.

18. Composition selon la revendication 17, dans laquelle l'agent oxydant est choisi parmi le peroxyde d'hydrogène, le peroxyde d'urée, les bromates ou ferricyanures de métaux alcalins, les persels, les enzymes d'oxydoréduction éventuellement en présence de leur donneur ou cofacteur respectif.

19. Procédé de teinture directe des fibres kératiniques, **caractérisé en ce qu'**une composition selon l'une quelconque des revendications 1 à 18 est appliquée sur les fibres kératiniques pendant un temps suffisant pour développer la coloration désirée.

20. Dispositif à plusieurs compartiments dans lequel un premier compartiment contient une composition telle que définie à l'une quelconque des revendications 1 à 16 et un deuxième compartiment contient un agent oxydant.

21. Utilisation pour la teinture directe des fibres kératiniques d'une composition telle que définie à l'une quelconque des revendications 1 à 18.

22. Utilisation d'un polymère amphotère tel que défini dans l'une quelconque des revendications 1 à 11, dans une composition de teinture directe des fibres kératiniques afin de réduire le tachage du cuir chevelu.

## Claims

1. Composition for the direct dyeing of keratinous fibres comprising, in a medium appropriate for dyeing, at least one direct dye and at least one amphoteric polymer comprising the repetition of:
(i) one or more units resulting from a monomer of acrylamide type,
(ii) one or more units resulting from a monomer of dialkyldiallylammonium halide type, and
(iii) at least 50 mol% of one or more units resulting from a monomer of vinylcarboxylic acid type.

2. Composition according to Claim 1, in which the units resulting from a monomer of acrylamide type are units with the following structure (I): 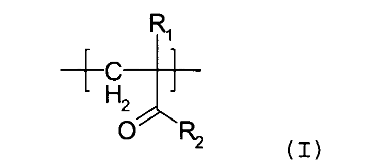 in which :
- R₁ denotes H or CH₃,
- R₂ is chosen from an amino, dimethylamino, tert-butylamino, dodecylamino or -NH-CH₂OH radical.

3. Composition according to Claim 2, in which R₁ denotes H and R₂ is an amino radical.

4. Composition according to any one of the preceding claims, in which the units resulting from a monomer of dialkyldiallylammonium halide type are units with the following structure (II): 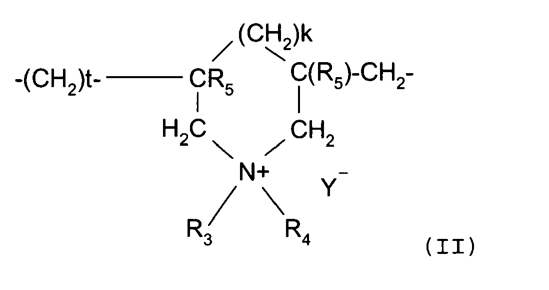 in which:
- k and t are equal to 0 or 1, the sum k + t being equal to 1;
- R₅ denotes a hydrogen atom or a methyl radical;
- R₃ and R₄ denote, independently of one another, an alkyl group having from 1 to 4 carbon atoms, a hydroxyalkyl group in which the alkyl group has from 1 to 5 carbon atoms, or an amido (C₁-C₄) alkyl group, or R₃ and R₄ form, jointly with the nitrogen atom to which they are attached, heterocyclic groups;
- Y⁻ is a bromide, chloride, acetate, borate, citrate, tartrate, bisulphate, bisulphite, sulphate or phosphate anion.

5. Composition according to Claim 4, in which the units resulting from the monomer of dialkyldiallylammonium halide type are units of the formula (II) in which R₅ denotes a hydrogen atom and R₃ and R₄ denote a methyl radical, Y⁻ denoting a chloride anion.

6. Composition according to any one of the preceding claims, in which the units resulting from a monomer of vinylcarboxylic acid type are chosen from the units of formula (III): 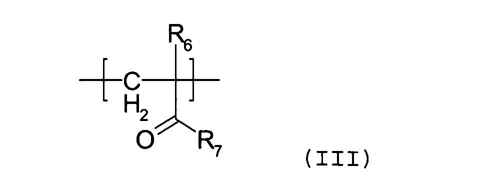 in which :
- R₆ denotes H or CH₃,
- R₇ denotes a hydroxyl radical or an -NH-C(CH₃)₂-CH₂-SO₃H radical.

7. Composition according to Claim 6, in which the unit resulting from a monomer of vinylcarboxylic acid type is that for which R₆ denotes a hydrogen atom and R₇ denotes a hydroxyl radical.

8. Composition according to any one of the preceding claims, in which the amphoteric polymer or polymers comprise from 50 to 90 mol% of units resulting from a monomer of vinylcarboxylic acid type.

9. Composition according to any one of the preceding claims, in which the amphoteric polymer or polymers comprise from 1 to 40 mol% of units resulting from a monomer of acrylamide type.

10. Composition according to any one of the preceding claims, in which the amphoteric polymer or polymers comprise from 1 to 40 mol% of units resulting from a monomer of dialkyldiallylammonium halide type.

11. Composition according to any one of the preceding claims, in which the amphoteric polymer or polymers are chosen from acrylamide/ dimethyldiallylammonium chloride/acrylic acid terpolymers comprising at leat 50 mol% of units resulting from acrylic acid.

12. Composition according to any one of the preceding claims, in which the amphoteric polymer or polymers are present in an amount of between 0.1 and 10% by weight, with respect to the total weight of the composition.

13. Composition according to any one of the preceding claims, in which the direct dye or dyes are chosen from acid, basic or neutral direct dyes.

14. Composition according to Claim 13, in which the direct dye or dyes are chosen from neutral, acid or cationic nitrobenzene direct dyes, neutral, acid or cationic azo, azomethine or methine direct dyes, neutral, acid or cationic quinone direct dyes, azine direct dyes, triarylmethane direct dyes, indoamine direct dyes and natural direct dyes.

15. Composition according to any one of the preceding claims, comprising at least one cationic direct dye carrying a permanent charge in the molecule.

16. Composition according to any one of the preceding claims, comprising a total amount of direct dye(s) ranging from 0.001% to 20% by weight, with respect to the total weight of the composition.

17. Composition according to any one of the preceding claims, comprising at least one oxidizing agent.

18. Composition according to Claim 17, in which the oxidizing agent is chosen from hydrogen peroxide, urea hydrogen peroxide, alkali metal bromates, alkali metal ferricyanides, persalts or oxidation-reduction enzymes, optionally in the presence of their respective donor or cofactor.

19. Method for the direct dyeing of keratinous fibres, **characterized in that** a composition according to any one of Claims 1 to 18 is applied to keratinous fibres for a time sufficient to develop the desired colouring.

20. Multicompartment device, in which a first compartment comprises a composition as defined in any one of Claims 1 to 16 and a second compartment comprises an oxidizing agent.

21. Use for the direct dyeing of keratinous fibres of a composition as defined in any one of Claims 1 to 18.

22. Use of an amphoteric polymer as defined in any one of Claims 1 to 11 in a composition for the direct dyeing of keratinous fibres in order to reduce staining of the scalp.

## Patentansprüche

1. Zusammensetzung für die Direktfärbung von Keratinfasern, die in einem zum Färben geeigneten Medium mindestens einen Direktfarbstoff und mindestens ein amphoteres Polymer enthält, das die folgenden Wiederholungseinheiten aufweist:
(i) eine oder mehrere Einheiten, die von einem Monomer vom Typ Acrylamid stammen,
(ii) eine oder mehrere Einheiten, die von einem Monomer vom Typ eines Dialkyldiallylammoniumhalogenids stammen,
(iii) mindestens 50 Mol-% einer oder mehrerer Einheiten, die von einem Monomer vom Typ einer Vinylcarbonsäure stammen.

2. Zusammensetzung nach Anspruch 1, wobei die Einheiten, die von einem Monomer vom Acrylamidtyp stammen, Einheiten der folgenden Struktur (I) sind: 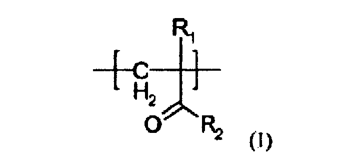 worin
- R₁ H oder CH₃ bedeutet, und
- R₂ unter Amino, Dimethylamino, *tert*-Butylamino, Dodecylamino oder -NH-CH₂OH ausgewählt ist.

3. Zusammensetzung nach Anspruch 2, wobei R₁ H bedeutet und R₂ eine Aminogruppe ist.

4. Zusammensetzung nach einem der vorhergehenden Ansprüche, wobei die Einheiten, die von einem Monomer vom Typ Dialkyldiallylammoniumhalogenid stammen, Einheiten der folgenden Struktur (II) sind: 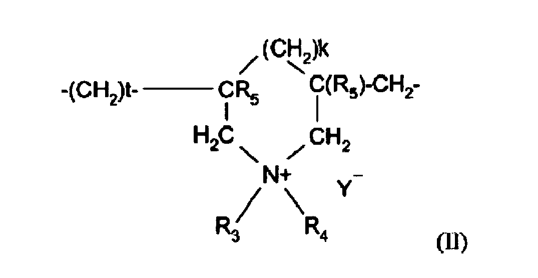 in der Formel:
- k und t sind 0 oder 1, wobei die Summe k + t 1 bedeutet;
- R₅ bedeutet ein Wasserstoffatom oder die Methylgruppe;
- R₃ und R₄ bedeuten unabhängig voneinander eine Alkylgruppe mit 1 bis 4 Kohlenstoffatomen, eine Hydroxyalkylgruppe, bei der die Alkylgruppe 1 bis 5 Kohlenstoffatome aufweist, oder eine Amidoalkyl(C₁₋₄)gruppe oder R₃ und R₄ bilden gemeinsam mit dem Stickstoffatom, an das sie gebunden sind, heterocyclische Gruppen;
- Y- bedeutet Bromid, Chlorid, Acetat, Borat, Citrat, Tartrat, Bisulfat, Bisulfit, Sulfat oder Phosphat.

5. Zusammensetzung nach Anspruch 4, wobei die Einheiten, die von einem Monomer vom Typ Dialkyldiallylammoniumhalogenid stammen, Einheiten der Struktur (II) sind, worin R₅ ein Wasserstoffatom bedeutet und R₃ und R₄ Methyl bedeuten, wobei Y-Chlorid ist.

6. Zusammensetzung nach einem der vorhergehenden Ansprüche, wobei die Einheiten, die von einem Monomer vom Typ einer Vinylcarbonsäure stammen, unter den Einheiten der Formel (III) ausgewählt sind: 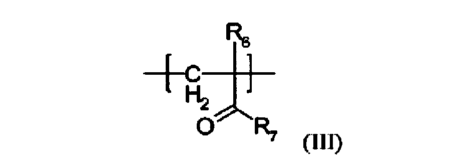 in der Formel:
- R₆ bedeutet H oder CH₃,
- R₇ bedeutet die Hydroxygruppe oder eine Gruppe -NH-C(CH₃)₂-CH₂-SO₃H.

7. Zusammensetzung nach Anspruch 6, bei der die Einheit, die von einem Monomer vom Typ einer Vinylcarbonsäure stammt, eine Einheit ist, bei der R₆ ein Wasserstoffatom bedeutet und R₇ die Hydroxygruppe ist.

8. Zusammensetzung nach einem der vorhergehenden Ansprüche, wobei das oder die amphotere(n) Polymer(e) 50 bis 90 Mol-% Einheiten enthalten, die vom einem Monomer vom Typ einer Vinylcarbonsäure stammen.

9. Zusammensetzung nach einem der vorhergehenden Ansprüche, wobei das oder die amphotere(n) Polymer(e) 1 bis 40 Mol-% Einheiten enthalten, die von einem Monomer vom Typ Acrylamid stammen.

10. Zusammensetzung nach einem der vorhergehenden Ansprüche, wobei das oder die amphotere(n) Polymer(e) 1 bis 40 Mol-% Einheiten enthalten, die vom einem Monomer vom Typ Dialkyldiallylammoniumhalogenid stammen.

11. Zusammensetzung nach einem der vorhergehenden Ansprüche, wobei das oder die amphotere(n) Polymer(e) unter den Acrylamid/Dimethyldiallylammoniumchlorid/Acrylsäure-Terpolymeren ausgewählt sind, die mindestens 50 Mol-% Einheiten enthalten, die von Acrylsäure stammen.

12. Zusammensetzung nach einem der vorhergehenden Ansprüche, wobei das oder die amphotere(n) Polymer(e) in einem Mengenanteil im Bereich von 0,1 bis 10 Gew.-%, bezogen auf das Gesamtgewicht der Zusammensetzung, enthalten sind.

13. Zusammensetzung nach einem der vorhergehenden Ansprüche, wobei der oder die Direktfarbstoff(e) unter den sauren, basischen oder neutralen Direktfarbstoffen ausgewählt sind.

14. Zusammensetzung nach Anspruch 13, bei der der oder die Direktfarbstoff(e) unter den neutralen, sauren oder kationischen nitrierten direktziehenden Benzolfarbstoffen, neutralen, sauren oder kationischen direktziehenden Azofarbstoffen, Azomethin-Farbstoffen oder Methin-Farbstoffen, neutralen, sauren oder kationischen direktziehenden Chinonfarbstoffen, direktziehenden Azinfarbstoffen, direktziehenden Triarylmethanfarbstoffen, direktziehenden Indoaminfarbstoffen und direktziehenden natürlichen Farbstoffen ausgewählt sind.

15. Zusammensetzung nach einem der vorhergehenden Ansprüche, die mindestens einen kationischen Direktfarbstoff enthält, der eine permanente Ladung im Molekül besitzt.

16. Zusammensetzung nach einem der vorhergehenden Ansprüche, die eine Gesamtmenge an Direktfarbstoff(en) im Bereich von 0,001 bis 20 Gew.-%, bezogen auf das Gesamtgewicht der Zusammensetzung, enthält.

17. Zusammensetzung nach einem der vorhergehenden Ansprüche, die mindestens ein Oxidationsmittel enthält.

18. Zusammensetzung nach Anspruch 17, wobei das Oxidationsmittel unter Wasserstoffperoxid, Harnstoffperoxid, Alkalimetallbromaten, Alkalimetallferricyaniden, Salzen von Persäuren und Redoxenzymen gegebenenfalls in Gegenwart ihres jeweiligen Donors oder Cofaktors ausgewählt ist.

19. Verfahren für die Direktfärbung von Keratinfasern, **dadurch gekennzeichnet, dass** eine Zusammensetzung nach einem der Ansprüche 1 bis 18 während einer Zeitspanne, die ausreichend ist, um die gewünschte Färbung zu bilden, auf die Keratinfasern aufgebracht wird.

20. Vorrichtung mit mehreren Abteilungen, bei der eine erste Abteilung eine Zusammensetzung, wie sie einem der Ansprüche 1 bis 16 definiert ist, und eine zweite Abteilung ein Oxidationsmittel enthält.

21. Verwendung einer Zusammensetzung, wie sie einem der Ansprüche 1 bis 18 definiert ist, für die Direktfärbung von Keratinfasern.

22. Verwendung eines amphoteren Polymers, wie es in einem der Ansprüche 1 bis 11 definiert ist, in einer Zusammensetzung für die Direktfärbung von Keratinfasern, um die Verfärbung auf der Kopfhaut einzuschränken.
